# FASCICULE DE BREVET EUROPEEN

(11) **EP 1 532 997 B1**
(45) Date de publication et mention de la délivrance du brevet: **29.03.2017**
(21) Numéro de dépôt: 04292750.9
(22) Date de dépôt: 22.11.2004
(51) Int. Cl.: A61M 5/32, A61M 5/24, A61M 5/315

(54) **Dispositif de protection d'aiguille destinè à une carpule**
Nadelschutzvorrichtung für eine Karpulle
Needle protecting device for a carpule

(30) Priorité: 24.11.2003 FR 0313757
(43) Date de publication de la demande: 25.05.2005
(73) Titulaire: SEDAT, 69540 Irigny (Rhône) (FR)
(72) Inventeur: Pessin, Olivier, 69290 Grezieu la Varenne (FR)
(74) Mandataire: Lavoix

(56) Documents cités:
- FR-A- 2 837 107
- US-A- 5 360 410
- US-A- 5 380 296
- US-A- 5 891 104
- US-A- 5 989 226
- US-A1- 2002 068 921

## Description

La présente invention concerne un dispositif de protection d'aiguille du type comportant :
- un support de protecteur délimitant une chambre de réception d'un réservoir de fluide à injecter équipé d'un piston, lequel support est équipé de moyens de solidarisation audit réservoir ;
- un protecteur d'aiguille mobile par rapport au support de protecteur, entre une position rétractée et une position déployée de protection de l'aiguille.

De nombreuses substances médicamenteuses sont conditionnées dans des carpules. Une carpule est formée d'un corps généralement cylindrique dont une extrémité présente un goulot rétréci obturé par une membrane perforable.

Le liquide à injecter est retenu dans le corps de la carpule par un piston coulissant.

Pour procéder à l'injection, la carpule est équipée d'une aiguille et d'un dispositif d'actionnement. A cet effet, la carpule est placée généralement dans un dispositif d'auto-injection comportant un corps dans lequel la carpule est reçue. Un ressort assure l'actionnement du piston de la carpule.

Par ailleurs, il est connu d'équiper les seringues d'injection de dispositifs de protection d'aiguille permettant de recouvrir l'aiguille à l'issue de l'injection afin d'éviter des piqûres accidentelles depuis l'extrémité de l'aiguille lors des manipulations de la seringue.

Les dispositifs de protection d'aiguille sont rapportés sur un corps de seringue. Ces dispositifs sont généralement solidarisés au corps de seringue par enclenchement élastique du dispositif sur une collerette d'appui des doigts prévue dans la partie arrière de la seringue.

Or, les carpules sont dépourvues de tels profils à leurs extrémités arrière de sorte que la mise en place d'un dispositif de protection d'aiguille sur une carpule est délicate.

L'invention a pour but de proposer un dispositif de protection d'aiguille pouvant être adaptée sur une carpule.

Un dispositif selon le préambule de la revendication 1 est connu du document US2002/0068921. A cet effet, l'invention a pour objet un dispositif auxiliaire de protection d'aiguille tel que définit dans la revendication 1.

Suivant d'autres caractéristiques de ce dispositif, prises isolément ou selon toutes les combinaisons techniquement possibles :
- le protecteur d'aiguille comporte une collerette extérieure permettant l'appui des doigts du praticien pour la réalisation d'une injection ;
- il comporte un poussoir initialement lié au support de protecteur ou au protecteur, lequel poussoir délimite une cavité de réception initiale de l'extrémité d'injection de l'aiguille et comporte une extrémité de connexion à un piston mobile de la carpule ;
- ledit poussoir est venu de matière avec le protecteur d'aiguille ;
- le poussoir est emboîté sur le support de protecteur ; et
- le support de protecteur et le protecteur d'aiguille comportent des moyens complémentaires d'enclenchement élastique propres à assurer la retenue du protecteur d'aiguille dans sa position déployée.

L'invention a en outre pour objet un dispositif d'injection comportant, d'une part, une carpule qui comprend, un corps tubulaire présentant à une extrémité distale un goulot, et un piston d'injection monté à coulissement dans le corps, et d'autre part, un dispositif de protection d'aiguille tel que décrit ci-dessus.

L'invention sera mieux comprise à la lecture de la description qui va suivre, donnée uniquement à titre d'exemple et faite en se référant aux dessins sur lesquels :
- la figure 1 est une vue éclatée en perspective d'un dispositif d'injection selon l'invention avant assemblage ;
- la figure 2 est une vue en élévation du dispositif de protection appartenant au dispositif d'injection de la figure 1, en position rétractée ;
- la figure 3 est une vue en coupe selon le plan III-III indiqué sur la figure 2 ;
- la figure 4 est une vue de dessus selon la flèche IV indiquée sur la figure 2 ;
- la figure 5 est une vue en coupe selon le plan indiqué V-V sur la figure 4 ;
- la figure 6 est une vue suivant le même plan de coupe que celui de la figure 3, du dispositif d'injection après mise en place d'une carpule et avant mise en place du poussoir ;
- la figure 7 est une vue analogue à celle de la figure 5, le dispositif d'injection étant en cours d'utilisation ;
- la figure 8 est une vue suivant le même plan de coupe que celui de la figure 5, du dispositif d'injection en fin d'injection ;
- la figure 9 est une vue identique à celle de la figure 8, la carpule du dispositif d'injection n'étant pas représentée ;
- la figure 10 est une vue en coupe selon le plan X-X indiquée sur la figure 8 ;
- les figures 11 à 13 illustrent le dispositif d'injection en position déployée, la figure 11 étant une vue analogue à celle de la figure 8, la figure 12 étant une vue en coupe selon le plan XII-XII indiqué sur la figure 11 et la figure 13 est une vue identique à celle de la figure 11, la carpule n'étant pas représentée ; et
- la figure 14 est une vue identique à celle de la figure 6 d'une variante de réalisation d'un dispositif selon l'invention.

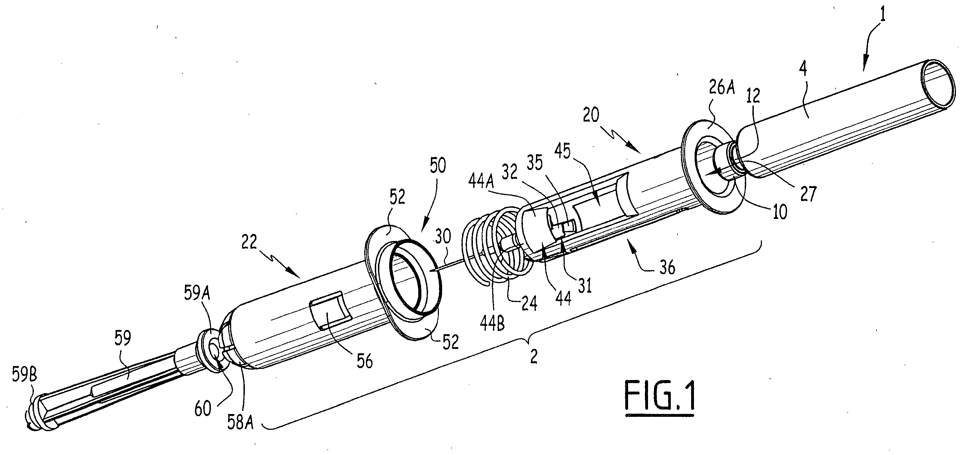

Sur la figure 1 sont représentés en perspective une carpule 1 et un ensemble de protection 2. Dans toute la suite, les termes « proximal » et « arrière » sont synonymes, tout comme les termes « distal » et « avant ».

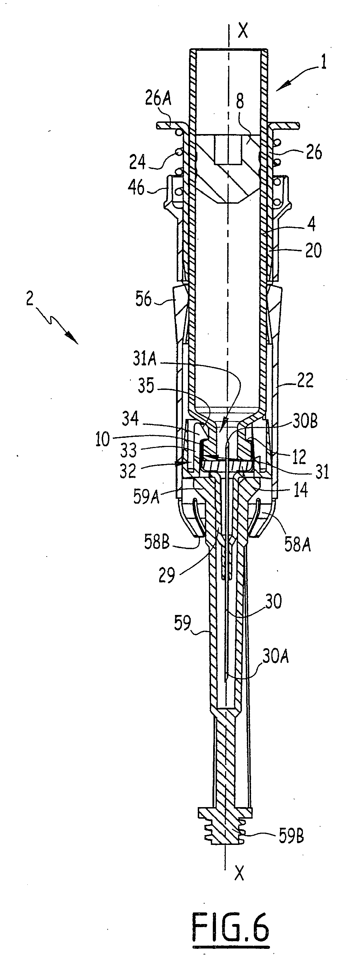

La carpule 1 est une carpule en verre de forme standard, destinée à un usage unique. Elle contient un liquide à injecter, de façon intramusculaire ou sous-cutanée, à un patient. Elle comporte à cet effet essentiellement un corps cylindrique de révolution 4 et un piston coulissant 8 visible sur la figure 6.

Le corps 4 de la carpule comporte, dans sa partie distale, un goulot rétréci 10 délimitant en arrière un épaulement circonférentielle 12. Le goulot 10 est obturé par un opercule perforable 14. L'opercule 14 est maintenu sur le goulot par une virole métallique sertie.

Sur l'essentiel de sa longueur et à son extrémité proximale, le corps 4 est cylindrique de section circulaire et est dépourvu de protubérance extérieure.

L'ensemble de protection 2, d'axe général X-X, comporte essentiellement, comme représenté sur la figure 1 :
- un support 20 de forme générale tubulaire ;
- un manchon protecteur 22 disposé coaxialement au support 20 et de diamètre supérieur à celui du support 20, et
- un ressort 24.

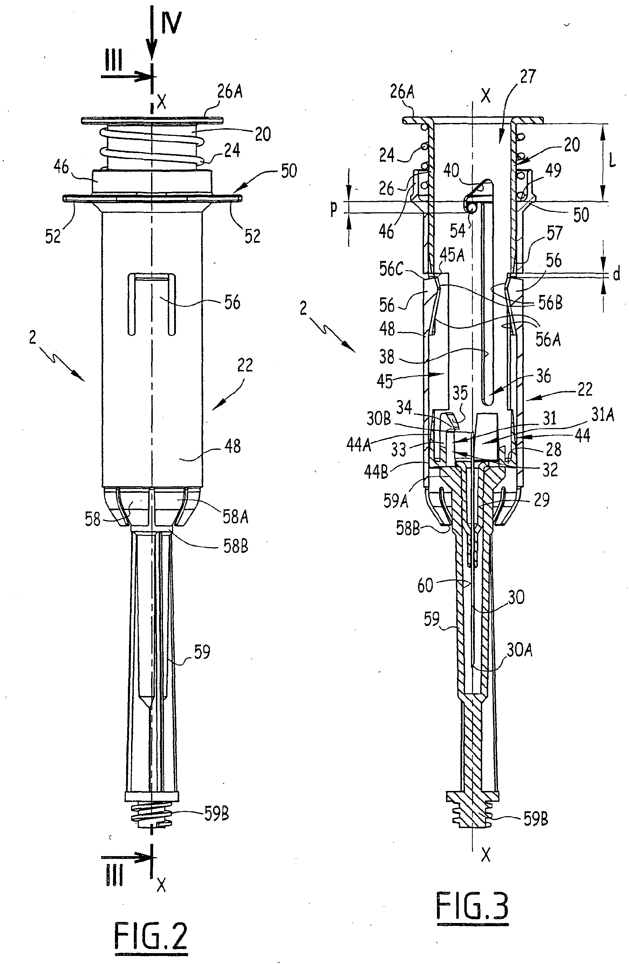

Ces trois éléments vont successivement être détaillés ci-dessous, en regard des figures 2 à 4.

Le support 20 comporte un tronçon principal 26, sensiblement cylindrique et délimitant une chambre 27 de réception de la carpule 1. Cette chambre a un diamètre interne sensiblement égal au diamètre externe du corps 4 de la carpule. Ce tronçon 26 présente à son extrémité proximale une collerette extérieure 26A formant une surface d'appui pour le ressort 24.

A son extrémité distale, le tronçon 26 du support est obturé par une paroi transversale 28 d'appui du goulot de la carpule. La paroi 28 présente généralement la forme d'un disque et délimite, en son centre, un passage pour une aiguille d'injection. La paroi 28 est prolongée axialement par un collet 29 de support d'une aiguille creuse d'injection 30. Le collet 29 fait saillie à l'extérieur de la chambre 27 suivant l'axe du support 20. L'extrémité libre du collet 29 est resserrée sur l'aiguille creuse 30 pour assurer sa retenue par collage, ou surmoulage.

L'aiguille 30 présente une extrémité d'injection 30A disposée à l'extérieur du support et une extrémité de perforation de la carpule 30B faisant saillie à l'intérieur de la chambre 27 suivant l'axe de celle-ci. Ainsi, l'extrémité 30B s'étend en arrière de la paroi 28.

Par ailleurs, le dispositif de protection comporte des moyens 31 de solidarisation du support 20 sur la carpule 1. Ces moyens 31, visibles notamment sur la figure 6 comportent des crochets 32 déformables élastiquement propres à s'enclencher élastiquement autour du goulot 10 de la carpule, en arrière de l'épaulement 12, en confinant le goulot 10 dans une cage 31A délimitée entre les crochets et la paroi transversale 28.

Ces crochets sont venus de matière avec le support 20. Ils sont par exemple au nombre de deux et sont répartis autour de l'axe longitudinal du support. Ils présentent chacun une jambe 33 s'étendant généralement parallèlement à l'axe de l'aiguille 30. Chaque jambe 33 est solidarisée à une extrémité à la paroi 28 et présente, à son autre extrémité, une saillie 34 tournée vers l'axe du support. Cette saillie 34 est propre à s'engager en arrière de l'épaulement 12 délimitée par le goulot 10 de la carpule.

Avantageusement, la saillie 34 de chaque crochet présente une rampe inclinée 35 tournée vers l'extrémité proximale du support, les rampes 35 convergeant l'une vers l'autre en direction de l'aiguille 30.

Deux rainures traversantes 36 en forme de crosse dont l'une est visible sur la figure 3 sont ménagées l'une en face de l'autre dans le tronçon principal 26. Chaque rainure est constituée d'une première partie rectiligne 38 qui s'étend sensiblement suivant l'axe X-X du support 20 sur une longueur supérieure à celle de l'aiguille 30, et d'une seconde partie rectiligne 40 qui s'étend de manière inclinée par rapport au même axe X-X. La partie inclinée 40 débouche à l'extrémité proximale de la première partie rectiligne 38, en formant un V dont la pointe est dirigée du côté proximal de l'ensemble 2.

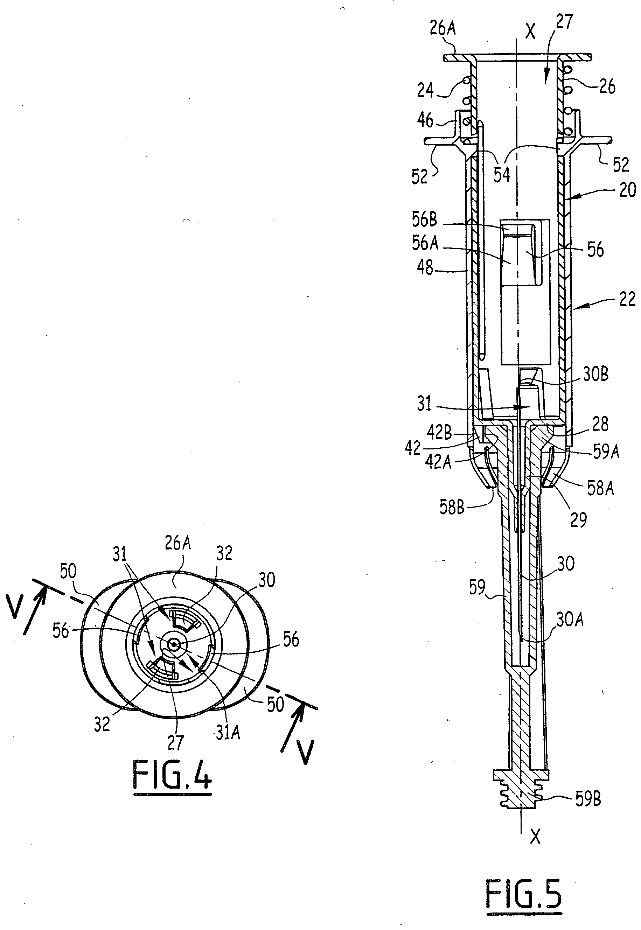

Le tronçon principal 26 comporte à son extrémité distale, au-delà de la paroi transversale 28, une première paire de languettes élastiques 42 diamétralement opposées, situées chacune dans le prolongement des rainures 36 (figure 5). Ces languettes 42 présentent une face interne 42A de forme sensiblement cylindrique et une face externe 42B sensiblement tronconique, divergente vers l'arrière.

Le tronçon principal 26 comporte une paire de rampes externes 44 diamétralement opposées, situées entre les languettes élastiques 42 en suivant la circonférence de l'extrémité distale de ce tronçon. Elles présentent une surface externe inclinée 44A sensiblement tronconique, divergente vers l'avant, et une surface distale 44B sensiblement cylindrique.

Des évidements longitudinaux 45 sont ménagés dans le tronçon principal 26 (figure 3) à l'extrémité proximale de ces rampes 44. Les surfaces externes 44A sont ainsi tournées vers les évidements 45.

Le manchon protecteur 22 est de longueur sensiblement égale à celle du corps 4 de la carpule 1. Il est constitué de deux tronçons cylindriques 46 et 48, le tronçon proximal 46 étant de diamètre légèrement supérieur à celui du tronçon principal 48. Ces deux tronçons se raccordent en formant un épaulement radial 49 d'appui du ressort 24.

Le manchon 22 comporte solidairement, dans sa partie proximale, une collerette extérieure 50 sous forme de deux oreilles 52 diamétralement opposées (figure 4).

Egalement dans sa partie proximale, mais à l'intérieur du manchon protecteur 22, deux tétons 54 diamétralement opposés sont solidaires du manchon (figure 5). Ces deux tétons sont reçus et guidés dans les deux rainures 36 du support 20. Le support et le manchon sont ainsi mobiles l'un par rapport à l'autre en translation suivant leur axe commun et en rotation limitée autour du même axe lorsque les tétons se trouvent dans les parties inclinées 40. Les parties inclinées 40 forment alors des poches de rétention des tétons 54, ces poches ayant une profondeur de rétention notée p sur la Figure 3. Cette profondeur est mesurée suivant l'axe du protecteur.

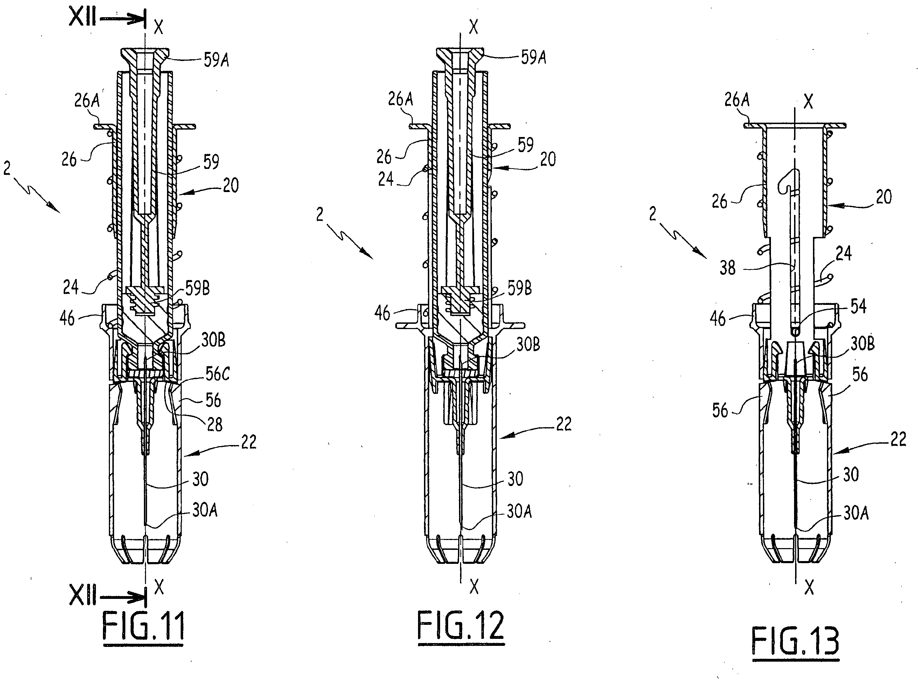

Plus précisément, le support 20 et le manchon 22 sont mobiles entre une position rétractée du manchon, dans laquelle l'essentiel du manchon recouvre l'essentiel du support et les tétons 54 sont situés à l'extrémité distale de chacune des parties de rainures inclinées 40, comme représenté sur les figures 2 à 7, et une position déployée du manchon dans laquelle celui-ci s'étend axialement en saillie du support et les tétons sont situés à l'extrémité distale de la partie de rainure rectiligne 38, comme représenté sur les figures 11 à 13.

Lorsque la carpule 1 est fixée sur l'ensemble 2, ces positions extrêmes correspondent respectivement à une configuration d'injection dans laquelle l'aiguille 30 est dégagée et destinée à être insérée dans un patient, et à une configuration de protection dans laquelle cette aiguille est entourée du manchon protecteur 22.

La partie proximale du manchon 22 comporte en outre intérieurement une paire de crochets longitudinaux déformables 56 diamétralement opposés. Ces crochets sont délimités dans le manchon 22 par des fentes latérales. Ces crochets sont liés au manchon à leur extrémité distale. Chaque crochet présente à son extrémité libre proximale une protubérance intérieure.

En l'absence de carpule, et comme illustré sur la Figure 3, les surfaces externes des crochets 56 s'étendent dans le prolongement du manchon. Au contraire, les protubérances internes de ces crochets font saillie à l'intérieur du passage cylindrique délimité par le manchon 22. Chaque protubérance présente une face avant 56A de forme sensiblement tronconique s'écartant vers l'avant de l'axe du manchon 22. Ces faces avant 56A sont ainsi tournées vers l'extrémité avant du protecteur.

Chaque face avant 56A est adaptée pour coopérer avec une surface inclinée 44A formée par les rampes 44 du support.

En outre, chaque protubérance intérieure présente une face arrière inclinée 56B tournée vers l'extrémité arrière du protecteur, et en particulier vers les deux oreilles 52. Ces surfaces 56B sont généralement tronconiques et divergent l'une de l'autre en direction de l'extrémité arrière du protecteur.

Le support 20 comporte des rampes tronconiques 57 disposées à l'arrière des évidements 45. Ces rampes sont inclinées vers les évidements 45 et sont adaptées pour coopérer avec les faces inclinées arrières 56B.

A son extrémité libre, chaque crochet 56 présente un front transversal incliné 56C formant butée.

En l'absence de carpule, tel qu'illustré sur la Figure 3, le front 56C de chaque crochet s'étend immédiatement en regard de l'extrémité correspondante de l'évidement 45 définissant une surface d'appui 45A pour le front 56C. La distance d séparant la face d'extrémité 56C du bord de l'évidement est inférieure à la profondeur p de la poche de rétention formée de la partie de rainure inclinée 40 dans laquelle est reçu le pion 54.

Dans la position rétractée du manchon, les crochets 56 s'étendent à l'intérieur des évidements 45 ménagés dans le support 20. Dans la position déployée du manchon, comme représenté sur la figure 11, les faces d'extrémité 56C des crochets 56 sont en butée axiale contre la paroi transversale 28, les crochets et languettes formant de la sorte un ensemble de blocage rigide en position déployée.

Le manchon 22 est par ailleurs pourvu à son extrémité distale d'une couronne tronconique 58 de diamètre progressivement décroissant vers son extrémité libre.

Dans ce mode de réalisation, la couronne tronconique 58 est formée d'une couronne de languettes déformables élastiquement 58A dont les bords distaux forment une ouverture 58B sensiblement circulaire.

A son extrémité libre distale, le manchon protecteur 22 est prolongé initialement par un poussoir 59 permettant l'actionnement du piston de la carpule. Ce poussoir 59 est constitué d'une tige présentant à son extrémité arrière une collerette 59A d'appui du pouce et à son extrémité avant ou distale, un filetage 59B propre à coopérer avec un taraudage complémentaire ménagé dans la face exposée du piston 8.

Le diamètre de l'ouverture 58B, lorsque les languettes sont au repos, est inférieur au diamètre de la collerette 59A du poussoir.

Le poussoir 59 est creux et délimite un logement 60 s'ouvrant axialement au centre de la collerette 59A d'appui du pouce. Le logement 60 reçoit initialement l'extrémité 30A de l'aiguille 30, comme illustré sur les figures 3 et 6. Ainsi, le poussoir 59 forme initialement un capuchon de protection de l'aiguille.

La forme du logement 60, au voisinage de son ouverture, est complémentaire à celle du collet 29, de sorte que, comme illustré sur la figure 3, le collet 29 s'emboîte exactement dans le logement 60. Pour assurer une retenue du poussoir 59 sur le support 20. Une nervure est ménagée dans la paroi interne délimitant le logement 20, alors qu'une collerette complémentaire fait saillie sur le collet 29 pour assurer un enclenchement élastique. Dans cette position, la collerette 59A du poussoir est appliquée contre la paroi 28 et s'étend entre cette paroi et les languettes 58A du manchon protecteur.

Le ressort 24 est un ressort spiralé, disposé entre le manchon protecteur 22 et le support de protecteur 20. Plus précisément, le ressort est logé entre la collerette 26A du support et l'épaulement 49 du manchon 22. Dans la position rétractée du manchon, le ressort 24 est dans un état comprimé, possédant ainsi une énergie de décompression liée à la raideur du ressort et à la différence entre la longueur du ressort à l'état au repos et sa longueur, notée L sur les figures 2 à 7, à l'état comprimé. Cela revient à dire que le ressort 24 présente un seuil de force de compression supplémentaire qui correspond à la force minimale nécessaire pour comprimer davantage le ressort à partir de son état comprimé initial des figures 2 à 7. La raideur du ressort et/ou la longueur de compression initiale L sont choisies de telle sorte que ce seuil de force soit supérieur à l'effort de poussée nécessaire pour déplacer le piston 8 de la carpule 1 sur toute sa course d'injection. Plus précisément, la force du ressort à l'état verrouillé est supérieure à la somme de l'effort d'injection, c'est-à-dire d'évacuation du liquide à l'extérieur de l'aiguille 30, et des efforts de décollement et de glissement du piston 8 à l'intérieur du corps 4 de la carpule.

Le fonctionnement du dispositif d'injection selon l'invention est le suivant :
Le dispositif de protection 2 est assemblé dans sa configuration rétractée, c'est-à-dire celle des figures 2 à 7. Le manchon protecteur 22 est pour cela enfilé autour du support 20 à partir de l'extrémité distale du support, en disposant entre eux le ressort 24. Plus précisément, on déplace axialement vers l'arrière le manchon par rapport au support, tout en déformant radialement vers l'extérieur les crochets 56 au moyen d'un outil adapté, pour qu'ils atteignent axialement la partie avant des évidements longitudinaux 45. Puis toujours en déplaçant le manchon vers l'arrière, les pions 54 sont appliqués contre les surfaces externes 42B des languettes 42, jusqu'à ce que les pions soient reçus dans les parties de rainures 38. Le déplacement du protecteur 22 vers l'arrière se poursuit ensuite jusqu'à ce que les tétons 54 soient reçus dans les parties de rainures inclinées 40, en faisant pivoter l'un par rapport à l'autre le support et le protecteur. Le protecteur se retrouve ainsi en position rétractée. L'outil est enfin retiré.

Dans cette position, et telle qu'illustré sur la Figure 3, les protubérances intérieures des crochets 56 font saillie à l'intérieur du passage de réception de la carpule de sorte que la surface transversale arrière 56C s'étend immédiatement en avant du bord correspondant de l'évidement 45.

Le front d'extrémité forme une butée propre à coopérer avec le bord des évidements 45 pour éviter un recul du manchon protecteur 22 vers l'extrémité arrière du support 20. Les crochets 56 sont alors en position de blocage.

Comme il sera expliqué ultérieurement, dans la mesure où la retenue du ressort assurant le déplacement du manchon protecteur est libérée par déplacement du protecteur vers l'arrière, tout risque de déclenchement accidentel du manchon protecteur lors du transport du dispositif de protection est évité puisque le manchon protecteur est bloqué dans sa direction de libération de manière indépendante des moyens de retenue du ressort, tant qu'aucune carpule n'est introduite dans le support de protecteur.

La carpule 1 est préremplie d'un liquide à injecteur à un patient.

La carpule est insérée à l'intérieur de l'ensemble 2 pour former le dispositif d'injection, tel que représenté sur la Figure 6. Plus précisément, le corps 4 de la carpule est déplacé sensiblement axialement à l'intérieur du support 20. Lors de l'engagement du corps 4 de la carpule dans le support 20, et comme illustré sur la Figure 6, le goulot 20 s'engage entre les saillies 34 des crochets 32 en prenant appui d'abord sur les rampes inclinées 35. Lors de l'enfoncement du goulot 10, les extrémités libres des crochets 32 s'écartent permettant l'engagement du goulot entre les crochets. Lorsque les saillies 34 ont franchi l'épaulement 12 du goulot, les crochets se redressent élastiquement, provoquant l'engagement des saillies 34 en arrière de l'épaulement 12 et le confinement du goulot 10 dans la cage 31A définie entre les saillies 34 des crochets et la paroi transversale 28.

La carpule est alors immobilisée axialement par rapport au support 20.

Lors de l'engagement du goulot entre les crochets, l'opercule 14 est perforé par l'aiguille creuse 30, depuis l'extrémité 30B de celle-ci.

Ainsi, le liquide contenu dans la carpule est en contact avec l'aiguille creuse et peut s'écouler au travers de celle-ci.

Lorsque le praticien est prêt à réaliser l'injection du liquide contenu dans la carpule, il détache le poussoir 59 en le tirant axialement vers l'avant.

Lors de son engagement, les languettes 58A se déforment élastiquement pour permettre le passage de la collerette 59A. Elles reprennent ensuite leur position initiale après passage de la collerette 59A, de sorte que le poussoir ne peut plus être remis en place après un retrait initial, les languettes inclinées 58A interdisant le passage de la collerette 59A jusque dans l'espace délimité entre le support 20 et le manchon protecteur 22.

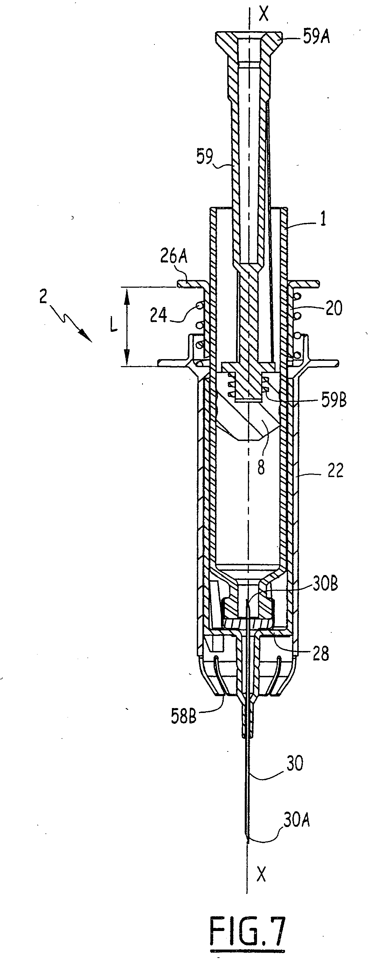

Après que le poussoir 59 a été détaché, celui-ci est vissé depuis son extrémité filetée 59B dans le piston 8. Il s'étend alors comme illustré sur la figure 7. Dans cette configuration, l'extrémité d'injection 30A de l'aiguille est exposée.

Le praticien pique ensuite l'aiguille 30 dans un patient. Il injecte le liquide contenu dans la carpule en exerçant une poussée sur le poussoir 59 relié au piston 8, son index et son majeur demeurant en contact avec les faces dirigées vers l'aiguille des oreilles 52. Durant l'injection, aucun mouvement ne se produit entre le support de protecteur 20 et le manchon protecteur 22, le ressort 24 demeurant comprimé avec une longueur L, comme représenté sur la figure 7.

L'injection se poursuit jusqu'à ce que le poussoir 59 et le piston 8 parviennent en fin de course d'injection.

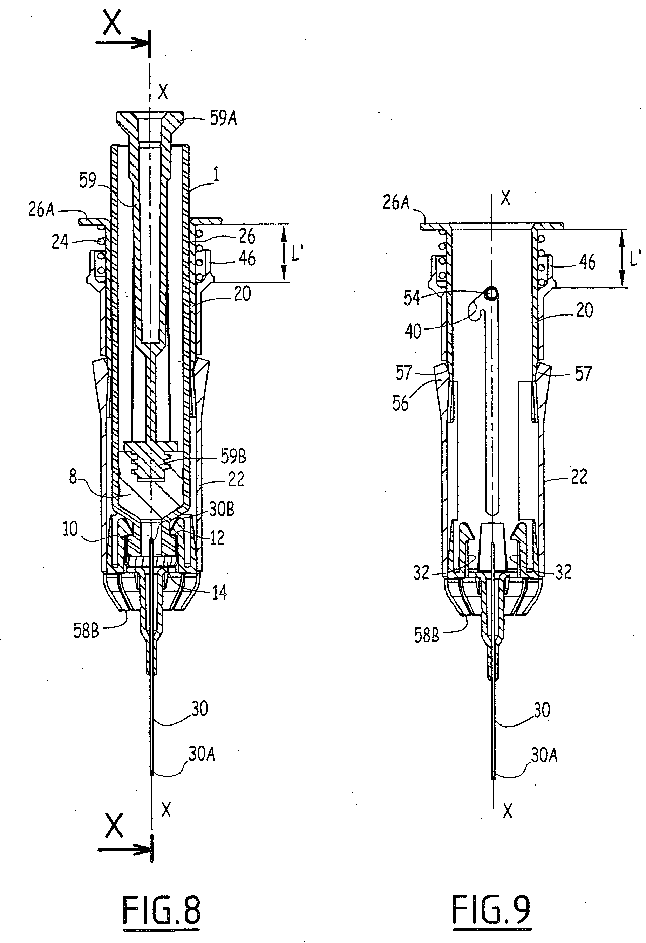

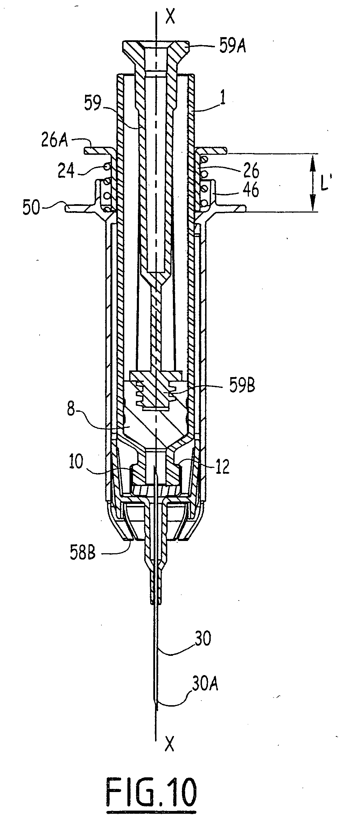

Le praticien retire alors l'aiguille du patient. Pour déclencher l'ensemble de protection 2, le praticien exerce une pression supplémentaire sur le poussoir 59 et le piston 8. Cette pression doit être supérieure à la force prédéterminée produite par le ressort 24 à l'état verrouillé, de sorte que ce ressort est davantage comprimé et passe de sa longueur L à une longueur L' plus petite, comme représenté sur les figures 8 à 10. Pour ce faire, en raisonnant à support de carpule immobile, le manchon protecteur 22 se déplace axialement vers l'extrémité proximale du support 20. Le praticien réalise ce mouvement en exerçant une pression correspondante au moyen de son index et de son majeur sur les oreilles 52 de la collerette 50 du manchon 22. Cette pression entraîne, de façon combinée au mouvement de translation, la rotation du manchon protecteur 22 autour du support de carpule 20, les tétons 54 étant guidés par les parties de rainure inclinées 40. Ce mouvement de rotation se poursuit jusqu'à ce que les tétons atteignent l'extrémité proximale de cette partie de rainure 40, c'est-à-dire l'extrémité proximale de la partie de rainure longitudinale 38, comme visible sur la figure 9. Le dispositif 2 est alors en position de déverrouillage du ressort 24.

Le mouvement du protecteur d'aiguille vers l'extrémité arrière du support suivant la direction de libération du ressort est rendu possible par la coopération des faces inclinées arrières 56B des protubérances des crochets et des rampes 57. Lors du mouvement du protecteur, une déformation accrue des crochets vers l'extérieur se produit comme illustré sur la Figure 8. Ainsi, les crochets s'écartent l'un de l'autre pour passer au-dessus de la surface extérieure du manchon protecteur au-delà des évidements 45.

Le praticien relâche ensuite la pression qu'il exerçait jusqu'alors sur la collerette 50, permettant au ressort 24 de se détendre jusqu'à un état de repos. Les tétons 54 se déplacent en translation à l'intérieur de la partie de rainure longitudinale 38, jusqu'à l'extrémité distale de celle-ci comme représenté sur la figure 13. Le mouvement de translation du manchon protecteur 22 par rapport au support 20 est contrôlable par le praticien, si ce dernier relâche progressivement la retenue digitale qu'il exerce la collerette 50. Lorsque les tétons 54 sont arrivés à l'extrémité distale de la rainure 36 (figure 13), le protecteur est dans sa position déployée.

Par ailleurs, lorsque le manchon protecteur est en mouvement de translation par rapport au support 20, les crochets 56 empruntent les évidements longitudinaux 45 du support, jusqu'à ce qu'il glissent le long des rampes distales 44 du support, par coopération de leurs surfaces complémentaires 56A et 44A.

En position déployée du protecteur, les crochets 56 sont maintenus par coopération des surfaces 56C et 28B, de sorte que le manchon protecteur 22 ne peut pas être ramené dans sa position initiale. De même, le manchon 22 ne peut pas être facilement arraché du support 20 puisque les tétons 54 sont en butée contre le fond distal de la partie de rainure longitudinale 38 (figure 13).

Le dispositif d'injection selon l'invention est ainsi simple d'utilisation, tout en permettant au praticien de contrôler le mouvement de recouvrement de l'aiguille par le manchon protecteur. Le nombre de pièces dont est constitué l'ensemble de protection 2 représenté est réduit à trois.

Le dispositif selon l'invention est adaptable à différents types de carpules, tant au niveau des formes qu'à celui des volumes. Ce dispositif présente donc l'avantage de ne pas remettre en cause la forme générale des carpules utilisées et par conséquent n'entraîne aucune modification des procédés industriels de remplissage de ces carpules.

Diverses variantes du dispositif selon l'invention sont envisageables ; les tétons 54 et/ou la collerette 50 du manchon protecteur 22 peuvent être rapportés sur le manchon 22 et non réalisés d'un seul tenant avec celui-ci.

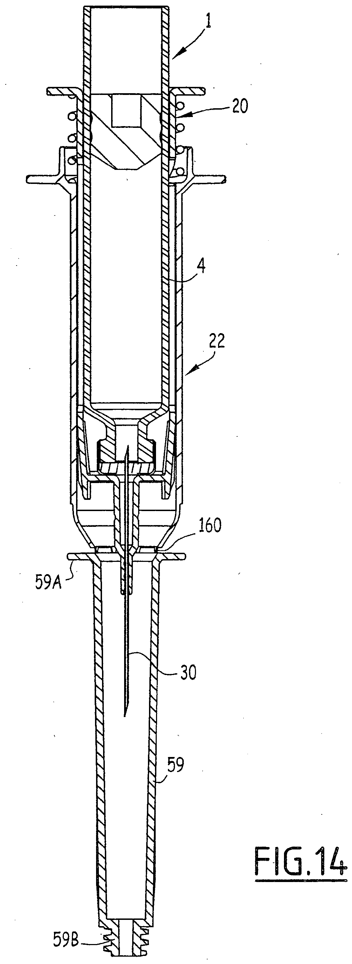

Comme illustré par la variante de la figure 14, le poussoir 59 est venu de matière avec le manchon protecteur 22 et est lié à ce dernier par des liens frangibles et notamment des plots frangibles 160.

## Revendications

1. Dispositif (2) auxiliaire de protection d'aiguille (30) comportant :
- un support de protecteur (20) délimitant une chambre (27) de réception d'un réservoir (1) de fluide à injecter équipé d'un piston (8), lequel support (20) est équipé de moyens de solidarisation (31) audit réservoir (1) ;
- un protecteur d'aiguille (22) mobile par rapport au support de protecteur (20), entre une position rétractée et une position déployée de protection de l'aiguille (30) ;
ledit dispositif (2) comportant
ladite aiguille (30), laquelle aiguille (30) est portée par ledit support de protecteur (20) suivant l'axe de la chambre (27) et présente une extrémité d'injection (30A) faisant saillie hors de la chambre (27) et une extrémité de perforation (30B) d'un opercule perforable du réservoir faisant saillie à l'intérieur de la chambre (27), ledit dispositif (2) comportant un ressort (24) interposé entre le support de protecteur (20) et le protecteur d'aiguille (22), lequel ressort (24) est initialement comprimé et est propre à solliciter le protecteur d'aiguille (22) vers sa position déployée, ledit dispositif (2) étant **caractérisé en ce que** lesdits moyens de solidarisation (31) comportent des crochets (32) faisant saillie dans ladite chambre (27) délimitant, autour de ladite aiguille (30), une cage (31) pour l'enclenchement élastique autour du goulot (10) d'un carpule (1) formant ledit réservoir, et
**en ce que** le support de protecteur (20) comporte, à son extrémité d'introduction de la carpule dans la chambre (27), une collerette (26A) d'appui du ressort (24).

2. Dispositif de protection d'aiguille selon la revendication 1, **caractérisé en ce que** le protecteur d'aiguille (22) comporte une collerette extérieure (50) permettant l'appui des doigts du praticien pour la réalisation d'une injection.

3. Dispositif de protection d'aiguille selon la revendication 1 ou 2, **caractérisé en ce qu'**il comporte un poussoir (59) initialement lié au support de protecteur (20) ou au protecteur (22), lequel poussoir (59) délimite une cavité (60) de réception initiale de l'extrémité d'injection (30A) de l'aiguille (30) et comporte une extrémité (59B) de connexion à un piston mobile (8) de la carpule (1).

4. Dispositif de protection d'aiguille selon la revendication 3, **caractérisé en ce que** ledit poussoir (59) est venu de matière avec le protecteur d'aiguille (22).

5. Dispositif selon la revendication 3, **caractérisé en ce que** le poussoir (59) est emboîté sur le support de protecteur (20).

6. Dispositif selon l'une quelconque des revendications précédentes, **caractérisé en ce que** le support de protecteur (20) et le protecteur d'aiguille (22) comportent des moyens complémentaires (42, 56) d'enclenchement élastique propres à assurer la retenue du protecteur d'aiguille (22) dans sa position déployée.

7. Dispositif selon l'une quelconque des revendications 1 à 6, **caractérisé en ce que** les crochets (32) sont venus de matière avec le support de protecteur (20).

8. Dispositif selon l'une quelconque des revendications 1 à 7, **caractérisé en ce que** le support de protecteur (20) comprend un tronçon principal (26) délimitant la chambre de réception (27) et comportant à son extrémité d'introduction de la carpule la collerette (26a) d'appui du ressort (24), le tronçon principal (26) étant obturé par une paroi transversale (28) d'appui du goulot d'une carpule (1), les crochets (32) étant repartis autour de l'axe de la chambre (27) et possédant chacun une jambe (33) s'étend généralement parallèlement à l'axe de la chambre (27), solidarisée à une extrémité à la paroi transversale (28) et présentant à son autre extrémité une saillie 34 tournée vers l'axe de la chambre (27) et propre à s'engager en arrière de l'épaulement délimité par le goulot d'une carpule (12).

9. Dispositif selon l'une quelconque des revendications 1 à 8, **caractérisé en ce que**, lors de l'insertion de la carpule (1) dans le support (20), les crochets sont adaptés pour que le goulot de la carpule (1) s'engage entre les crochets (32) de sorte que la carpule (1) est immobilisée axialement par rapport au support de protecteur (20), et un opercule de la carpule (1) est perforé par l'aiguille (30).

10. Dispositif d'injection comprenant, d'une part, une carpule (1) qui comprend un corps tubulaire (4) présentant un goulot rétréci (10) délimitant un épaulement (12) et équipé d'un opercule d'obturation perforable (14) et un piston d'injection (8) monté à coulissement dans le corps (4) et, d'autre part, un dispositif (2) de protection d'aiguille selon l'une quelconque des revendications précédentes.

## Patentansprüche

1. Hilfsvorrichtung (2) zum Nadelschutz (30), umfassend:
- einen Schutzträger (20), der eine Kammer (27) zur Aufnahme eines Behälters (1) von Fluid, das injiziert werden soll, begrenzt, die mit einem Kolben (8) ausgestattet ist, wobei der Träger (20) mit Mitteln zur festen Verbindung (31) an den Behälter (1) ausgestattet ist;
- einen Nadelschutz (22), der mit Bezug auf den Schutzträger (20) zwischen einer zurückgezogenen Position und einer ausgefahrenen Position zum Schutz der Nadel (30) beweglich ist;
- wobei die Vorrichtung (2) die Nadel (30) umfasst, wobei die Nadel (30) vom Schutzträger (20) gemäß der Achse der Kammer (27) getragen wird und ein Injektionsende (30A) aufweist, das aus der Kammer (27) hervorspringt, und ein Ende zur Perforation (30B) eines perforierbaren Deckels, das im Inneren der Kammer (27) hervorspringt, wobei die Vorrichtung (2) eine Feder (24) umfasst, die zwischen dem Schutzträger (20) und Nadelschutz (22) angeordnet ist, wobei die Feder (24) anfänglich komprimiert und dazu geeignet ist, den Schutz der Nadel (22) hin zu seiner ausgefahrenen Position vorzuspannen, wobei die Vorrichtung (2) **dadurch gekennzeichnet ist, dass** die Mittel zur festen Verbindung (31) Haken (32) umfassen, die aus der Kammer (27) hervorspringen, die um die Nadel (30) ein Gehäuse (31) begrenzen, um um den Hals (10) einer Ampulle (1) einzurücken, die den Behälter bildet, und dadurch, dass der Schutzträger (20) an einem Ende zur Einführung der Ampulle in die Kammer (27) einen Kragen (26A) zur Auflage der Feder (24) umfasst.

2. Vorrichtung zum Nadelschutz nach Anspruch 1, **dadurch gekennzeichnet, dass** der Nadelschutz (22) einen äußeren Kragen (50) umfasst, der die Auflage der Finger des Arztes zur Durchführung einer Injektion ermöglicht.

3. Vorrichtung zum Nadelschutz nach Anspruch 1 oder 2, **dadurch gekennzeichnet, dass** sie einen Schieber (59) umfasst, der anfänglich mit dem Schutzträger (20) oder mit dem Schutz (22) verbunden ist, wobei der Schieber (59) einen Hohlraum (60) zur anfänglichen Aufnahme des Injektionsendes (30A) der Nadel (30) begrenzt und ein Ende (59B) zur Verbindung mit einem beweglichen Kolben (8) der Ampulle (1) umfasst.

4. Vorrichtung zum Nadelschutz nach Anspruch 3, **dadurch gekennzeichnet, dass** der Schieber (59) einstückig mit dem Nadelschutz (22) ist.

5. Vorrichtung nach Anspruch 3, **dadurch gekennzeichnet, dass** der Schieber (59) auf dem Schutzträger (20) eingelassen ist.

6. Vorrichtung nach einem der vorhergehenden Ansprüche, **dadurch gekennzeichnet, dass** der Schutzträger (20) und der Nadelschutz (22) zusätzliche Mittel (42, 56) zum elastischen Einrücken umfassen, die ausgelegt sind, um den Rückhalt des Nadelschutzes (22) in seiner ausgefahrenen Position sicherzustellen.

7. Vorrichtung nach einem beliebigen der Ansprüche 1 bis 6, **dadurch gekennzeichnet, dass** die Haken (32) einstückig mit dem Schutzträger (20) sind.

8. Vorrichtung nach einem beliebigen der Ansprüche 1 bis 7, **dadurch gekennzeichnet, dass** der Schutzträger (20) einen Hauptabschnitt (26) umfasst, der die Aufnahmekammer (27) begrenzt, und an seinem Ende zur Einführung der Ampulle den Kragen (26a) zur Auflage der Feder (24) umfasst, wobei der Hauptabschnitt (26) durch eine Querwand (28) zur Auflage des Halses einer Ampulle (1) verschlossen ist, wobei die Haken (32) um die Achse der Kammer (27) verteilt sind und jeweils eine Strebe (33) aufweisen, die sich im Wesentlichen parallel zur Achse der Kammer (27) erstreckt, die an ein Ende der Querwand (28) fest verbunden ist und an ihrem anderen Ende einen Vorsprung (34) aufweist, der hin zur Achse der Kammer (27) gedreht und dazu geeignet ist, hinter dem Absatz einzugreifen, der vom Hals einer Ampulle (12) begrenzt ist.

9. Vorrichtung nach einem beliebigen der Ansprüche 1 bis 8, **dadurch gekennzeichnet, dass** bei der Einführung der Ampulle (1) in den Träger (20) die Haken so ausgelegt sind, dass der Hals der Ampulle (1) zwischen den Haken (32) eingreift, so dass die Ampulle (1) axial mit Bezug auf den Schutzträger (20) immobilisiert ist und ein Deckel der Ampulle (1) durch die Nadel (30) perforiert wird.

10. Injektionsvorrichtung, umfassend, einerseits, eine Ampulle (1), die einen rohrförmigen Körper (4) umfasst, der einen verengten Hals (10) umfasst, der einen Absatz (12) begrenzt und mit einem perforierbaren Verschlussdeckel (14) und einem Injektionskolben (8) ausgestattet ist, der gleitend im Körper (4) montiert ist, und andererseits eine Vorrichtung (2) zum Nadelschutz nach einem beliebigen der vorhergehenden Ansprüche.

## Claims

1. Auxiliary device (2) for protecting a needle (30) comprising:
- a protector support (20) delimiting a chamber (27) for receiving a reservoir (1) of fluid to be injected provided with a piston (8) and which support (20) is provided with means (31) for being fixedly joined to the reservoir;
- a needle protector (22) which can be moved relative to the protector support (20) between a retracted position and a deployed position for protecting the needle (30);
said device comprising:
said the needle (30), which needle (30) is carried by the protector support (20) in the axis of the chamber (27) and has an injection end (30A) protruding from the chamber (27) and a perforation end (30B) of a perforatable cap of the reservoir, protruding into the chamber (27), , said device comprising a spring interposed between the protector support (20) and the needle protector (22), which spring (24) is initially compressed and can urge the needle protector (22) towards its deployed position thereof,
said device being **characterized in that** the joining means (31) comprise hooks (32) protruding in the chamber (27) delimiting, around the needle (30), a cage (31) for resiliently latching around the neck (10) of a carpule (1) forming the reservoir, and **in that** the protector support (20) comprises, at the end thereof for introducing the carpule into the chamber (27), a collar (26A) for supporting the spring (24).

2. Needle protection device according to claim 1, wherein the needle protector (22) comprises an outer collar (50) allowing the fingers of the practitioner to be supported in order to carry out an injection.

3. Needle protection device according to claim 1 or 2, **characterized in that** it comprises a push-button (59) initially connected to the protector support (20) or the protector (22), which push-button (59) delimits a cavity (60) for initially receiving the injection end (30A) of the needle (30) and comprises an end (59B) for connecting to a moveable piston (8) of the carpule (1).

4. Needle protection device according to claim 3, **characterized in that** the push-button (59) is integral with the needle protector (22).

5. Device according to claim 3, **characterized in that** the push-button (59) is fitted to the protector support (20).

6. Device according to any one of the preceding claims, **characterized in that** the protector support (20) and the needle protector (22) comprise complementary resilient latching means (42, 56) which can bring about the retention of the needle protector (22) in the deployed position thereof.

7. Device according to any one of the preceding claims, **characterized in that** the hooks (32) are integral with the protector support (20).

8. Device according to any one of claims 1 to 7, **characterized in that** the protector support (20) comprises a main portion (26) delimiting the receiving chamber (27) and comprising at its end for introducing the carpule the collar (26A) for supporting the spring (24), the main portion (26) being closed off by a transverse wall (28) for supporting the neck of a carpule (1), the hooks (32) being distributed around the axis of the chamber (27) and each having a leg (33), which generally extends parallel with the axis of the chamber (27), fixedly joined at one end to the transverse wall (28) and having at its other end thereof a projection (34) directed towards the axis of the chamber (27) and adapted to engage behind the shoulder (12) delimited by the neck of a carpule (1).

9. Device according to any one of claims 1 to 8, **characterized in that**, when the carpule (1) is inserted into the support (20), the hooks are adapted so that the neck of the carpule (1) engages between the hooks (32) so that the carpule (1) is axially immobilised relative to the support (20), and a cap of the carpule (1) is perforated by the needle (30).

10. Injection device comprising, on the one hand, a carpule (1) which comprises a tubular body (4) which has a narrow neck (10) delimiting a shoulder (12) and which is provided with a perforatable closing cap (14) and an injection piston (8) mounted so as to be able to slide in the body (4) and, on the other hand, a needle protection device (2) according to any one of the preceding claims.
